# EUROPEAN PATENT APPLICATION

(11) **EP 4 571 758 A1**
(43) Date of publication of application: **18.06.2025**
(21) Application number: 23215440.1
(22) Date of filing: 11.12.2023
(51) Int. Cl.: G16H 20/10, A61B 5/00, A61M 16/00, G16H 10/60, G16H 20/40, G16H 40/63, G16H 50/20, G16H 50/30, G16H 50/70

(54) **ANESTHESIOLOGY INSTRUCTIONS GENERATED USING SUBJECT PHYSIOLOGICAL DATA AND SUBJECT METADATA**

(71) Applicant: Koninklijke Philips N.V., 5656 AG Eindhoven (NL)
(72) Inventor: MENA BENITO, Maria Estrella, Eindhoven (NL); VAN EE, Raymond, Eindhoven (NL); MENDOZA, Jose Luis Diaz, 5656AG Eindhoven (NL); WESTERINK, Joanne Henriëtte Desirée Monique, Eindhoven (NL)
(74) Representative: Philips Intellectual Property & Standards

(57) **Abstract**

Disclosed herein is a medical system (100, 300, 500) comprising a local memory (114) storing local machine executable instructions (116) and a control module (118). The control module is configured to generate anesthesiology instructions (124) in response to receiving subject physiological sensor data (120) and subject metadata (122) as input. The medical system further comprises a local computational system (108). Execution of the local machine executable instructions causes the computational system to: receive (202) the subject physiological sensor data via a network connection (110); receive (204) the subject metadata; receive (206) the anesthesiology instructions in response to inputting the subject physiological sensor data and the subject metadata into the control module; and provide (208) the anesthesiology instructions.

## Description

### TECHNICAL FIELD

The invention relates to medical communication and control of medical devices.

### BACKGROUND

Assessment of subject who will be undergoing a procedure involving anesthesia and is usually performed by an anesthesia team (nurse, allied healthcare, anesthesiologist).

The assessment follows a similar structure to other medical assessment, having the typical medical history together with the addition of anesthetic topics. The assessment includes information such as cardiovascular input (blood pressure, exercise tolerance, cardiovascular fitness), respiratory input (oxygenation, ventilation, obtrusive sleep apnea), gastro-esophageal reflux, drugs/medication records, and also past or anesthetic history, past surgical history, family/hereditary history.

### SUMMARY

The invention provides for a medical system, a method, and a computer program in the independent claims. Embodiments are given in the dependent claims.

In one aspect the invention provides for a medical system that comprises a local memory storing machine executable instructions and a control module. The control module is configured to generate anesthesiology instructions in response to receiving subject physiological sensor data and subject metadata as input. The medical system further comprises a local computational system. Execution of the local machine executable instructions causes the local computational system to receive subject physiological sensor data via a network connection. Execution of the local machine executable instructions further causes the local computational system to receive subject metadata. Execution of the local machine executable instructions further causes the local computational system to receive the anesthesiology instructions in response to inputting the subject physiological sensor data and the subject metadata into the control module. Execution of the local machine executable instructions further causes the local computational system to provide the anesthesiology instructions.

In another aspect, the invention provides for a method. The method comprises receiving subject physiological sensor data via a network connection. The method further comprises receiving subject metadata. The method further comprises receiving anesthesiology instructions in response to inputting the subject physiological sensor data and the subject metadata into a control module. The control module is configured to generate the anesthesiology instructions in response to receiving subject physiological sensor data and the subject metadata as input. The method further comprises providing the anesthesiology instructions.

In another aspect the invention provides for a computer program comprising machine executable instructions and a control module for execution by a local computational system. The control module is configured to generate anesthesiology instructions in response to receiving subject physiological sensor data and the subject metadata as input. Execution of the local machine executable instructions causes the local computational system to receive subject physiological sensor data via a network connection. Execution of the local machine executable instructions further causes the local computational system to receive subject metadata. Execution of the local machine executable instructions further causes the local computational system to receive the anesthesiology instructions in response to inputting the subject physiological sensor data and the subject metadata into the control module. Execution of the local machine executable instructions further causes the local computational system to provide the anesthesiology instructions.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of
Fig. 3 illustrates a further example of a medical system.
Fig. 4 shows a flow chart which illustrates a method of using the medical system of Fig. 3.
Fig. 5 illustrates a further example of a medical system.
Fig. 6 shows a flow chart which illustrates a method of using the medical system of Fig. 5.

### DESCRIPTION OF EMBODIMENTS

Like numbered elements in these figures are either equivalent elements or perform the same function. Elements which have been discussed previously will not necessarily be discussed in later figures if the function is equivalent.

A major disadvantage of current assessments for procedures using anesthesiology is that the subject comes to the location of the healthcare provider and the amount of data which is considered during the assessment may be limited. Examples may provide for a means of remotely assessing a subject and generating anesthesiology instructions from subject metadata and subject physiological sensor data.

In an example, the medical system comprises a local memory storing machine executable instructions and a control module. The control module is configured to generate anesthesiology instructions in response to receiving subject physiological sensor data and the subject metadata as input. The control module may take different forms in different examples. In some examples the control module may be a lookup table, an expert system, a trained machine learning module such as a neural network. The anesthesiology instructions may also take different forms in different examples. In one example, the anesthesiology instructions are configured for running or controlling or configuring pieces of medical equipment such as a medical imaging system and/or a robotic anesthesiology system. The subject physiological sensor data may also take different forms in different examples. In some examples it may be data which has been measured within a predetermined time that is descriptive of a subject, for example it may be current sensor data or the most recent sensor data, or it may be sensor data which has been accumulated historically or for a predetermined duration. The subject data may also contain different things in different examples. The subject data may for example be data which describes the physical characteristics of the subject as well as other details such as a desired medical procedure or need for a particular anesthesiology requirement.

The medical system further comprises a local computational system. Execution of the local machine executable instructions causes the local computational system to receive subject physiological sensor data via a network connection. The subject physiological sensor data is therefore acquired at a location that is remote or distinct to where the local computational system is. For example, the subject physiological sensor data could be received from a sensor package or other equipment being worn or adjacent to the subject. The subject physiological sensor data may also be obtained from a remote or home medical system. Execution of the local machine executable instructions further causes the local computational system to receive the subject metadata. The subject metadata may for example be received from a variety of locations such as retrieved from a local database or it may also be retrieved via the same network connection from the subject or from a database or data source associated with the subject. Execution of the local machine executable instructions further causes the local computational system to receive the anesthesiology instructions in response to inputting the subject physiological sensor data and the subject metadata into the control module. Execution of the local machine executable instructions further causes the local computational system to provide the anesthesiology instructions.

This example may be beneficial because it may provide for an improved means of generating the anesthesiology instructions. Currently, when anesthesiology instructions are generated or determined for a subject, the subject would likely travel to the hospital or other medical location and perform an interview with the anesthesiologist or other healthcare provider. This has the disadvantage that any measurements or data descriptive of the subject are only from this small time period as well as requiring the subject to travel to the same location as an anesthesiologist. The subject physiological sensor data may for example be acquired during the daily activity of the subject and may enable better data to be used for generating the anesthesiology instructions. This may for example have a better result in controlling other medical components or systems such as a medical imaging system or for providing better systems for controlling a robotically-operated anesthesiology system. Collecting the subject physiological sensor data from a remote location may also enable a larger and more diverse amount of subject physiological sensor data to be acquired.

In some examples, the control module may include one or more of the following functions or software components:
- Include feedback of an outcome functionality where a learning AI model could find the optimal weights of the input parameters to output the best anesthesia instructions.
- A clustering algorithm configured to profile the subject based on their input data, for example received via a user interface.
- An anomaly detection algorithm could detect abnormal values in the subject metadata and/or subject physiological sensor data when comparing an individual against a population of a group of subjects.
- An additional module could find out the more relevant factors / input parameters conditioning the anesthesia instructions after enough data entries have been collected.
- Other approaches can be used to extract the more relevant input factors such as decision tree / random forest methods.
- The AI learning model is configured to perform one or more of the following optimization / cost functions options:
   ∘ Minimize the risk (probability) of ending in ICU.
   ∘ Minimize the error in relevant vital signs from prediction and outcome.

In another aspect, the medical system further comprises a remote memory storing remote machine executable instructions. The medical system further comprises a subject sensor system configured for acquiring the subject physiological sensor data. The medical system further comprises a remote computational system. The combination of the remote memory, the subject sensor system and the remote computational system form components that are located at a position other than the local computational system. For this reason, they may then communicate via the network connection. Execution of the remote machine executable instructions further causes the remote computational system to acquire the subject physiological sensor data using the subject sensor system. Execution of the remote machine executable instructions further causes the remote computational system to transmit the subject physiological sensor data to the local computational system via the network connection. In some examples, the subject physiological sensor data is transmitted immediately or within a short or predetermined duration to the local computational system. In other examples, the subject physiological sensor data may be logged and in such cases, an archive or statistical analysis of the subject physiological sensor data may be transmitted to the local computational system via the network connection. However, in both cases, the subject physiological sensor data may for example be acquired as the subject is going through the subject's daily tasks as opposed to sitting in a physician's office. This may provide better or more accurate determination of the subject physiological sensor data and may result in more accurate or better generation of the anesthesiology instructions.

In another example the medical system further comprises a remote user interface controlled by the remote computational system. Execution of the remote machine executable instructions further causes the remote computational system to collect at least a portion of the subject metadata via the remote user interface. Execution of the remote machine executable instructions further causes the remote computational system to transmit the subject metadata to the local computational system via the network connection. A variety of subject metadata may be collected via the remote user interface. For example, questionnaires or data could be provided by the subject via the remote user interface. Such things as questionnaires or the results of questionnaires may also be provided. This may for example provide the subject physiological sensor data with additional things such as cognitive function or state of mind may also be objectively assessed and provided using the subject metadata. This may be advantageous because the portion of the subject metadata provided by the remote user interface may be correlated with or acquired at the same time as the subject physiological sensor data. For this reason, the subject metadata may be labeled with or correlated with the subject physiological sensor data. This may provide for a more accurate assessment of the current state of the subject.

In another example, execution of the remote computational instructions further causes the remote computational system to generate reference data descriptive of the subject metadata and/or the subject physiological sensor data. The reference data comprises any one of the following: an acquisition timestamp, location data descriptive of subject location during acquisition of the subject metadata and/or the subject physiological sensor data, accelerometer data descriptive of subject motion during acquisition of the subject metadata and/or the subject physiological sensor data, and combinations thereof. Execution of the remote computational instructions further causes the remote computational system to transmit the reference data to the local computational system via the network connection. The control module is further configured to receive as input the reference data when generating the anesthesiology instructions. This example may be particularly beneficial because the reference data provides a bridge for correlating or associating the subject physiological sensor data with locations, current physical activity between possibly the subject physiological sensor data and the subject metadata. This may therefore provide a larger context or provide a larger amount of data to provide a context so that the anesthesiology instructions may be generated more accurately or completely.

In another example the subject sensor system is a wearable device. This may for example include wrist wearable devices or other devices attached to the subject such as an insulin pump or insulin sensor. This may also include such things as blood oxygen sensors and other types of devices, which are able to make physiological measurements on the subject. Obtaining the data in this way may be particularly beneficial as opposed to obtaining it during a short examination during a medical interview.

In another example, the subject sensor system is a smartphone. This may be beneficial because people tend to typically carry their smartphone with them in a continuous fashion and this may provide for a large amount of subject physiological sensor data.

In another example, the subject sensor system is a portable computing device.

In another example, the subject sensor system is a vital sign measuring camera. For example, a camera within the room where the subject is located or even the camera from a smartphone or mobile phone may be used to monitor the subject. This may be useful for telling such things as pulse rate and other cardiovascular parameters.

In another example, the subject sensor system is a home medical system. A home medical system as used herein is a system located in the residence of a subject, which may be used for monitoring and/or helping a subject to maintain their personal health. This may include such things as an interface for exchanging information or data with a medical provider as well as providing for a suite of sensors for measuring relevant physiological properties of the subject. This may be beneficial because the home medical system may for example provide a repository of subject metadata and/or subject physiological sensor data which may be used to provide accurate data and an assessment for generating more accurate or relevant anesthesiology instructions.

In another example the subject physiological sensor data comprises electrocardiogram (ECG) data.

In another example the subject physiological sensor data comprises respiration rate data.

In another example the subject physiological sensor data comprises oxygen blood saturation level.

In another example the subject physiological sensor data comprises a heart rate.

In another example the subject physiological sensor data comprises blood pressure.

In another example the subject physiological sensor data comprises a weight. For example, the scale may be incorporated into a home medical system or a scale may be able to communicate with a smartphone or other device which is part of the subject sensor system.

In another example the subject physiological sensor data comprises a galvanic skin conductance measurement system.

In another example the medical system further comprises a robotic anesthesiology device. Execution of the local machine executable instructions further causes the computational system to output instructions adapted for controlling the robotic anesthesiology device using the anesthesiology instructions. In this example, the anesthesiology instructions contain commands which are used to control a robotic anesthesiology device. A robotic anesthesiology device, as used herein, encompasses an automatic device which is able to provide at least partially the demonstration of an anesthesiology agent or to assist a subject during such a procedure. For example, it may provide respiratory or breathing assistance. In other cases it may control a dispenser of agents or chemicals used for anesthesiology. This example may be beneficial because it may provide for a means of automatically configuring an automatic anesthesiology device using the best data available for a subject.

In another example, the medical system further comprises a magnetic resonance imaging system. The anesthesiology instructions further comprise magnetic resonance imaging control commands configured to adjust acquisition of the k-space data by the magnetic resonance imaging system. The local memory further stores pulse sequence commands configured to control the magnetic resonance imaging system to acquire the k-space data. Execution of the local machine executable instructions further causes the local computational system to adjust the configuration of the magnetic resonance imaging system using the anesthesiology instructions. For example, the pulse sequence commands can be adjusted to fit the anesthesiology instructions such as when substances may be administered to a subject or it may delay acquisition until after a certain period of time when, for example, the anesthesiology instructions have been executed by a robotic anesthesiology device. Execution of the local machine executable instructions further causes the local computational system to acquire the k-space data by controlling the magnetic resonance imaging system with the pulse sequence commands. The adjustment of the magnetic resonance imaging system by the anesthesiology plan may for example comprise/control triggering of the acquisition of the k-space data, adjusting the pulse sequence commands, and combinations thereof. This may be advantageous because it may provide for a means of automatically configuring the magnetic resonance imaging system when the anesthesiology instructions are provided or used.

In another example the medical system further comprises a local display controlled by the local computational system. Execution of the local machine executable instructions further causes the local computational system to render the anesthesiology instructions on the display. This may be useful in the case where an operator or physician is intended to follow the instructions. This may provide a convenient means of providing the anesthesiology instructions.

In another example the control module comprises a database system indexed using a subject profile. Execution of the local machine executable instructions further causes the local computational system to determine the subject profile by playing a statistical algorithm to the subject metadata. For example, particular key or average values may be applied to the subject metadata. In other examples such things as a clustering algorithm may be used to classify the subject profile. Execution of the local machine executable instructions further causes the local computational system to retrieve the anesthesiology instructions from the database system using the subject profile. In some cases the raw data of the subject profile may also be used to retrieve or index the data files in the database system.

In another example, execution of the local machine executable instructions further causes the computational system to receive manual control commands generated during execution of the anesthesiology instructions. Execution of the local machine executable instructions further causes the computational system to update the anesthesiology instructions in the subject database with the manual control commands if the manual control commands differ from the anesthesiology instructions by more than a predetermined criterion and these may be the anesthesiology commands retrieved using the subject profile. This example may be beneficial because if a physician or other operator needs to take manual control, then these values for the manual control can be used to update the database correctly.

In another example, the statistical algorithm is further configured for detecting an out of distribution condition when being applied to the subject metadata and the subject physiological sensor data. Execution of the local machine executable instructions further causes the local computational system to halt retrieval of anesthesiology instructions if the out of distribution condition is detected. For example, it may range check particular values of the subject metadata or the subject physiological sensor data. If either of these values are out of particular ranges then it may indicate that there is a special or noteworthy divergence from the expected values. In this case, the system may then be configured to automatically not provide the anesthesiology instructions. This may for example provide for improved safely.

In another example, the subject metadata comprises social history.

In another example, the subject metadata comprises lifestyle data.

In another example, the subject metadata comprises demographic data.

In another example, the subject metadata comprises age.

In another example, the subject metadata comprises weight.

In another example, the subject metadata comprises the type of procedure.

In another example, the subject metadata comprises electronic medical record data. For example, data may be retrieved from headers or data within a DICOM file.

In another example, the subject metadata comprises exercise tolerance.

In another example, the subject metadata comprises cardiovascular fitness.

In another example, the subject metadata comprises respiratory input.

In another example, the subject metadata comprises oxygenation.

In another example, the subject metadata comprises ventilation rate or level.

In another example, the subject metadata comprises the presence of obtrusive sleep apnea.

In another example, the subject metadata comprises the presence of gastro-esophageal reflux.

In another example, the subject metadata comprises drug use records.

In another example, the subject metadata comprises medical records.

In another example, the subject metadata comprises anesthetic or anesthesiology history.

In another example, the subject metadata comprises past surgical history.

In another example, the subject metadata comprises family history.

In another example, the subject metadata comprises genetic traits.

In another example the anesthesiology instructions comprise a pre-operative risk score and/or a post-operative risk score. This may for example be data assigned to particular anesthesiology commands and may be used to indicate a particular pre-operative or post-operative risk. This may for example be useful in deciding if the anesthesiology instructions should be followed or if for example it is desired to use manual control instead.

In an example the invention excludes surgical procedure performed or performing a surgical step in conjunction with the administration of anesthetics or other drugs according to the anesthesiology instructions and/or the administration of anesthetics or other drugs to the subject according to the anesthesiology instructions.

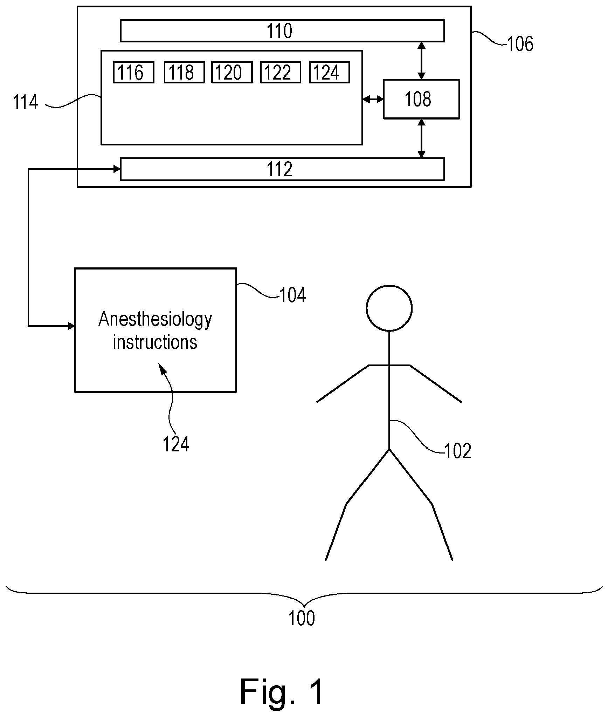

Fig. 1 illustrates an example of a medical system 100. In this example an operator 102 is looking at an optional display 104. The medical system 100 comprises a local computer 106. The local computer 106 comprises a local computational system 108. The local computer 106 further comprises a local hardware interface or local network interface 110 that is in communication with the local computational system 108. The local computer 106 is further shown as comprising a local user interface 112 that is also in communication with the local computational system 108. The display 104 is part of the local user interface 112.

The local computer 106 is further shown as comprising a local memory 114 that is also in communication with the local computational system 108. The local memory 114 is intended to represent various types of memory which may be accessible to the local computational system 108.

The local memory 114 is shown as containing local machine executable instructions 116 that enable the local computational system 108 to communicate with other computer systems via the local network interface 110 and/or also control other components. The local machine executable instructions 116 also enable the local computational system 108 to perform various data processing and numerical tasks. The local memory 114 is further shown as containing a control module 118. The local memory 114 is further shown as containing subject physiological sensor data 120 and subject metadata 122. The local memory 114 is further shown as storing anesthesiology instructions 124 that were received from the control module 118 in response to inputting the subject physiological sensor data 120 and the subject metadata 122 into the control module.

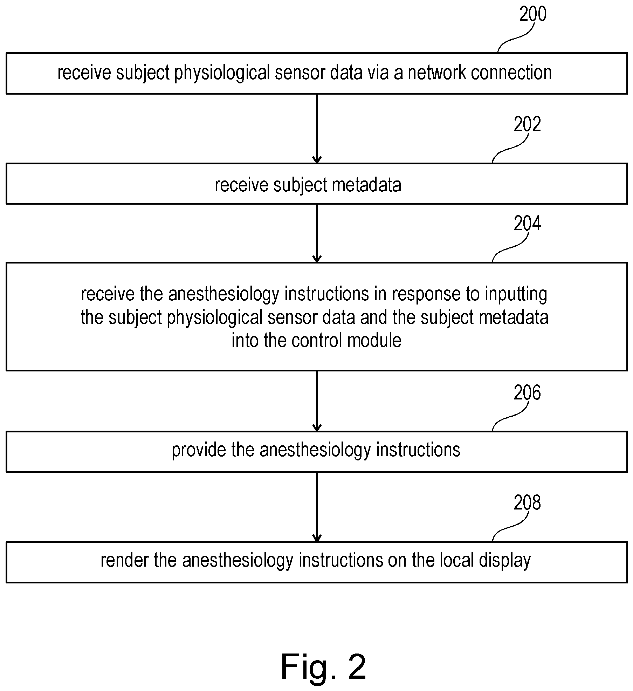

Fig. 2 shows a flowchart which illustrates a method of operating the medical system 100 of Fig. 1. In step 200, the subject physiological sensor data 120 is received via the network connection 110. In step 202, the subject metadata 122 is received. This may for example be stored locally in the local memory 114 or it may also be received via the local network interface 110. In step 204, the anesthesiology instructions 124 are received in response to inputting the subject physiological sensor data 120 and the subject metadata 122 into the control module 118. In step 206, the anesthesiology instructions 124 are provided. This may for example include using at least a portion of the anesthesiology instructions to control other medical devices and/or to display it on the display 104. In step 208, the anesthesiology instructions are optionally rendered on the local display 104.

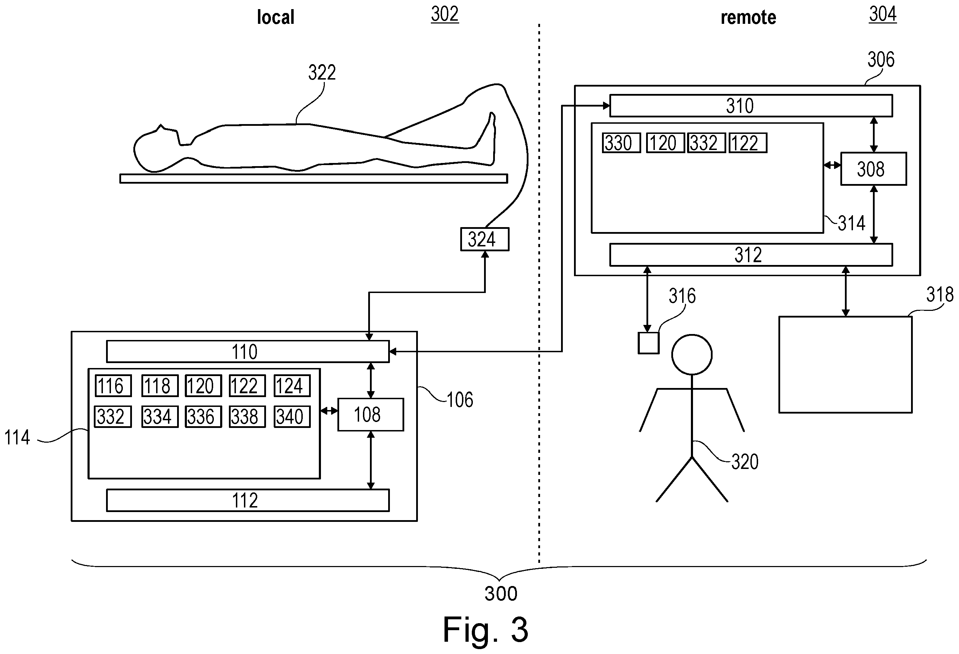

Fig. 3 illustrates a further example of a medical system 300. The medical system 300 is similar to the medical system 100 depicted in Fig. 1 except that it additionally comprises components at a remote 304 location. In Fig. 3 there are components located locally 302 and those located remotely 304.

The local computer system 106 is located at the local position 302. Additionally, there is a robotic anesthesiology device 324 which is able to perform an anesthesiology operation or function on the subject 322. There are two views of the subject; there is a local view 322 and a remote view 320. In the local view the subject 322 is using or is connected to the robotic anesthesiology device 324.

At the remote 304 location there is additionally a remote computer 306. The remote computer 306 comprises a remote computational system 308 connected to a remote network interface 110, a remote user interface 312, and a remote memory 314. Additionally, the remote user interface 318 is shown as being connected to a subject sensor system 316 and a remote user interface 318. The subject sensor system 316 is used to acquire the subject physiological sensor data 120. The remote user interface 318 may be used to enter at least a portion of the subject metadata 122. The portion of the metadata 122 and the subject physiological sensor data 120 are shown as being stored in the remote memory 314. These may be transferred via the network interfaces 310, 110 to the local memory 114. Additionally, reference data 332 that is descriptive of either the subject physiological sensor data 120 or the subject metadata 122 may also be generated or acquired by the remote computer system 306. This may also be transferred to the local memory 114.

The local memory 114 is further shown as containing a subject profile 334 that was generated using the subject physiological sensor data 120 and the subject metadata 122. The subject profile 334 is then used to query a database system 336 which may be part of the control module 118. This may be used to provide the anesthesiology instructions 124. The anesthesiology instructions 124 may be used to generate instructions 338 used for controlling the robotic anesthesiology device 324. During the course of operating the remote anesthesiology device 324 the system may be optionally overridden by a user. In this case, the manual control commands 340 may be recorded. These may be considered to be a correction to the instructions 338. The manual control commands 340 may then be used to correct or alter the record in the database system 336 referenced using the subject profile 334.

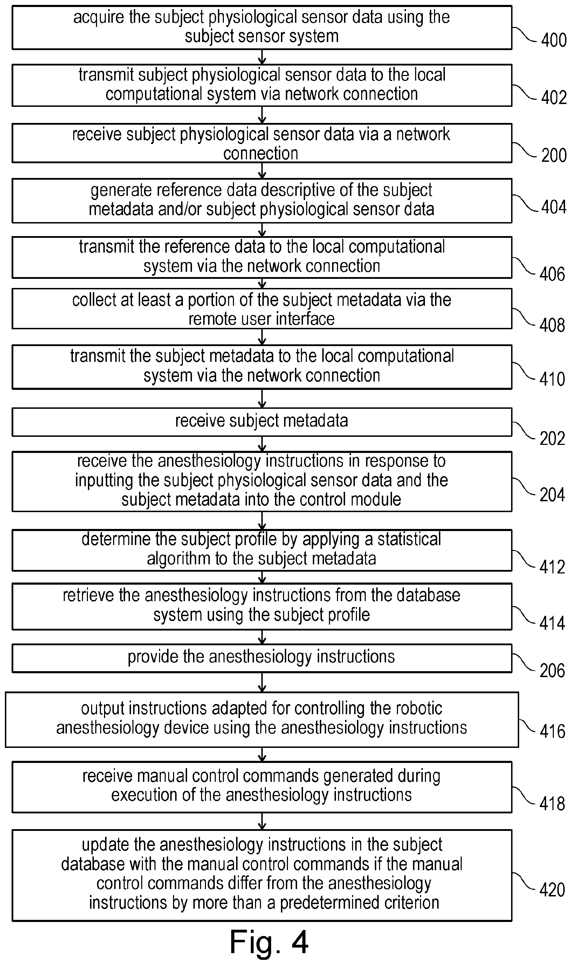

Fig. 4 shows a flowchart which illustrates a method of operating the medical system 300 of Fig. 3. In step 400, the subject physiological sensor data 120 is acquired using the subject sensor system 316. In step 402, the subject physiological sensor data 120 is transmitted to the local computational system 108 via the network connection 110. Step 200, as illustrated in Fig. 2, is performed. In step 404, the reference data 332 that is descriptive of the subject metadata 122 and/or the subject physiological sensor data 120 is generated by the remote computational system 308. In step 406, the reference data 332 is transmitted to the local computational system 108 via the network connection 110. In step 408, at least a portion of the subject metadata 122 is collected via the remote user interface 318. In step 410, the subject metadata 122 is transmitted to the local computational system 108 via the network connection 110. Steps 202 and 204 are performed as was illustrated in Fig. 2.

In step 412, the subject profile 334 is determined by applying a statistical algorithm to the subject metadata 122. In examples, the statistical algorithm could calculate the mean or average value of different parameters in the subject metadata or the subject physiological sensor data and this could be used to generate the subject profile. In other examples a clustering algorithm or a classification algorithm (such as a convolutional neural network) could be used to assign a classification which is then used as the subject profile.

In step 416, instructions 338 adapted for controlling the robotic anesthesiology device 324 are output using the anesthesiology instructions 124. For example, the anesthesiology instructions may be timing diagrams or timing information which is converted into detailed instructions or code which can be used to directly control the robotic anesthesiology device 324.

In step 418, manual control commands 340 are received or collected during execution of the anesthesiology instructions 338. This for example could be the manual control commands 340. In step 420, the anesthesiology instructions 338 are updated in the subject database for the record associated with the subject profile 334 using the manual control commands 340.

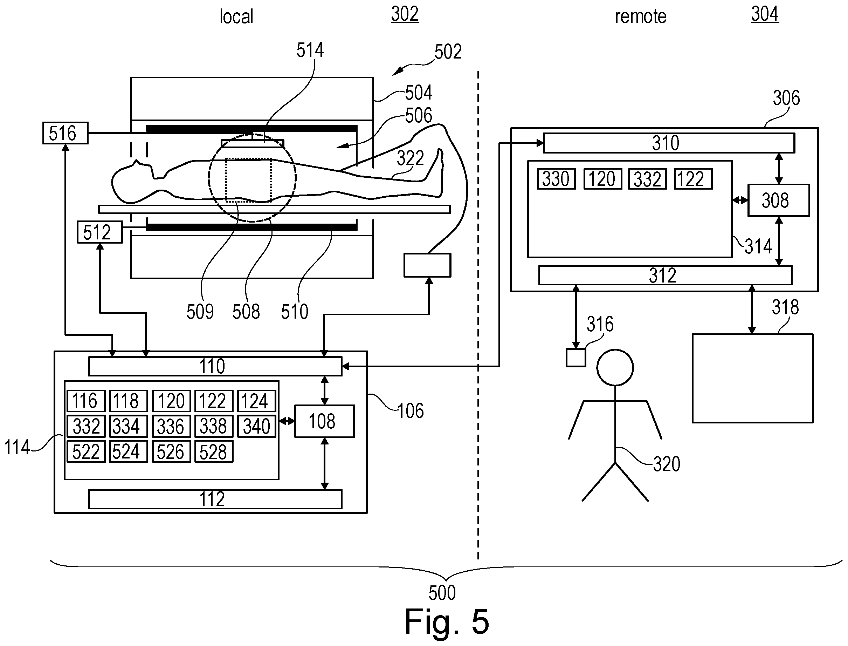

Fig. 5 illustrates a further example of a medical system 500. The medical system 500 depicted in Fig. 5 is similar to the medical system 300 depicted in Fig. 3 except that it additionally comprises a magnetic resonance imaging system 502.

The magnetic resonance imaging system 502 comprises a magnet 504. The magnet 504 is a superconducting cylindrical type magnet with a bore 506 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 506 of the cylindrical magnet 504 there is an imaging zone 508 where the magnetic field is strong and uniform enough to perform magnetic resonance imaging. A field of view 509 is shown within the imaging zone 508. The k-space data are acquired for the field of view 509. The region of interest could be identical with the field of view 509 or it could be a sub volume of the field of view 509. The subject 322 is shown as being supported by a subject support 520 such that at least a portion of the subject 322 is within the imaging zone 508 and the field of view 509.

Within the bore 506 of the magnet there is also a set of magnetic field gradient coils 510 which is used for the acquisition of measured k-space data to spatially encode magnetic spins within the imaging zone 508 of the magnet 504. The magnetic field gradient coils 510 are connected to a magnetic field gradient coil power supply 512. The magnetic field gradient coils 510 are intended to be representative. Typically, magnetic field gradient coils 510 contain three separate sets of coils for spatial encoding in three orthogonal spatial directions. A magnetic field gradient power supply supplies current to the magnetic field gradient coils. The current supplied to the magnetic field gradient coils 510 is controlled as a function of time and may be ramped or pulsed.

Adjacent to the imaging zone 508 is a radio frequency coil 514 for manipulating the orientations of magnetic spins within the imaging zone 508 and for receiving radio transmissions from spins also within the imaging zone 508. The radio frequency antenna may contain multiple coil elements. The radio frequency antenna may also be referred to as a channel or antenna. The radio frequency coil 514 is connected to a radio frequency transceiver 516. The radio frequency coil 514 and radio frequency transceiver 516 may be replaced by separate transmit and receive coils and a separate transmitter and receiver. It is understood that the radio frequency coil 514 and the radio frequency transceiver 516 are representative. The radio frequency coil 514 is intended to also represent a dedicated transmit antenna and a dedicated receive antenna. Likewise, the transceiver 516 may also represent a separate transmitter and receiver. The radio frequency coil 514 may also have multiple receive/transmit elements and the radio frequency transceiver 516 may have multiple receive/transmit channels. The transceiver 516 and the gradient controller 512 are shown as being connected to the hardware interface 106 of the computer system 102.

The memory 114 is further shown as containing pulse sequence commands 522 which can be used to control the magnetic resonance imaging system 502 to acquire k-space data 526. The anesthesiology instructions 124 may for example be used to control the timing of the operation of the magnetic resonance imaging system 502. This could for example be used to trigger the use of the pulse sequence commands 522 or even to modify the pulse sequence commands 522 into the modified pulse sequence commands 524. The memory 114 is shown as storing the k-space data 526 that was acquired either using the pulse sequence commands 522 or the modified pulse sequence commands 524. The memory 114 is further shown as containing a magnetic resonance image 528 reconstructed from the k-space data 526.

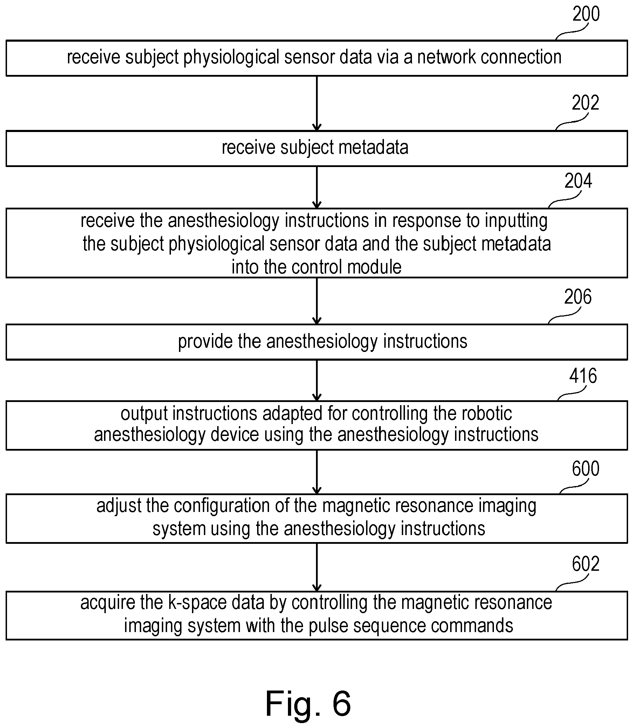

Fig. 6 shows a flowchart which illustrates a method of operating the medical system 500 of Fig. 5. Steps included in Fig. 4 may also be included or added to the method illustrated in Fig. 6. Steps 200, 202, 204, and 206 are performed as illustrated in Figs. 2 and 4. Step 416 is performed as was illustrated in Fig. 4. In step 600 the configuration of the magnetic resonance imaging system 502 is adjusted using the anesthesiology instructions 124. As was mentioned above, this could also include modifying or triggering the pulse sequence commands 522 or 524. In step 602 the k-space data 526 is acquired by controlling the magnetic resonance imaging system with the pulse sequence commands 522 or the modified pulse sequence commands 524.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: operator
- 104: display
- 106: local computer
- 108: local computational system
- 110: local hardware interface / local network interface
- 112: local user interface
- 114: local memory
- 116: local machine executable instructions
- 118: control module
- 120: subject physiological sensor data
- 122: subject metadata
- 124: anesthesiology instructions
- 200: receive subject physiological sensor data via a network connection
- 202: receive subject metadata
- 204: receive the anesthesiology instructions in response to inputting the subject physiological sensor data and the subject metadata into the control module
- 206: provide the anesthesiology instructions
- 208: render the anesthesiology instructions on the local display
- 300: medical system
- 302: local region
- 304: remote region
- 306: remote computer
- 308: remote computational system
- 310: remote network interface
- 312: remote user interface
- 314: remote memory
- 316: subject sensor system
- 318: remote user interface
- 320: fist view of subject
- 322: second view of subject
- 324: robotic anesthesiology device
- 330: remote machine executable instructions
- 332: reference data
- 334: subject profile
- 336: database system
- 338: instructions
- 340: manual control commands
- 400: acquire the subject physiological sensor data using the subject sensor system
- 402: transmit the subject physiological sensor data to the local computational system via the network connection
- 404: generate reference data descriptive of the subject metadata and/or the subject physiological sensor data
- 406: transmit the reference data to the local computational system via the network connection
- 408: collect at least a portion of the subject metadata via the remote user interface
- 410: transmit the subject metadata to the local computational system via the network connection
- 412: determine the subject profile by applying a statistical algorithm to the subject metadata
- 414: retrieve the anesthesiology instructions from the database system using the subject profile
- 416: output instructions adapted for controlling the robotic anesthesiology device using the anesthesiology instructions
- 418: receive manual control commands generated during execution of the anesthesiology instructions
- 420: update the anesthesiology instructions in the subject database with the manual control commands if the manual control commands differ from the anesthesiology instructions by more than a predetermined criterion
- 500: medical system
- 502: magnetic resonance imaging system
- 504: magnet
- 506: bore of magnet
- 508: imaging zone
- 509: field of view
- 510: magnetic field gradient coils
- 512: magnetic field gradient coil power supply
- 514: radio frequency coil
- 516: transceiver
- 520: subject support
- 522: pulse sequence commands
- 524: modified pulse sequence commands
- 526: k-space data
- 528: magnetic resonance image
- 600: adjust the configuration of the magnetic resonance imaging system using the anesthesiology instructions
- 602: acquire the k-space data by controlling the magnetic resonance imaging system with the pulse sequence commands

## Claims

1. A medical system (100, 300, 500) comprising:
- a local memory (114) storing local machine executable instructions (116) and a control module (118), wherein the control module is configured to generate anesthesiology instructions (124) in response to receiving subject physiological sensor data (120) and subject metadata (122) as input;
- a local computational system (108), wherein execution of the local machine executable instructions causes the computational system to:
- receive (202) the subject physiological sensor data via a network connection (110);
- receive (204) the subject metadata;
- receive (206) the anesthesiology instructions in response to inputting the subject physiological sensor data and the subject metadata into the control module; and
- provide (208) the anesthesiology instructions.

2. The medical system of claim 1, wherein the medical system further comprises:
- a remote memory (314) storing remote machine executable instructions (330);
- a subject sensor system (316) configured for acquiring the subject physiological sensor data; and
- a remote computational system (308), wherein execution of the remote machine executable instructions causes the remote computational system to:
- acquire (400) the subject physiological sensor data using the subject sensor system; and
- transmit (402) the subject physiological sensor data to the local computational system via the network connection.

3. The medical system of claim 2, wherein the medical system further comprises a remote user interface controlled by the remote computational system, wherein execution of the remote machine executable instructions further causes the remote computational system to:
- collect at least a portion of the subject metadata via the remote user interface; and
- transmit the subject metadata to the local computational system via the network connection.

4. The medical system of claim 3, wherein execution of the remote computational instructions further causes the remote computational system to:
- generate (404) reference data (332) descriptive of the subject metadata and/or the subject physiological sensor data, wherein the reference data comprises any one of the following: an acquisition time stamp, location data descriptive of a subject location during acquisition of the subject metadata and/or the subject physiological data, accelerometer data descriptive of subject motion during acquisition of the subject metadata and/or the subject physiological data, and combinations thereof; and
- transmit (406) the reference data to the local computational system via the network connection, wherein the control module is further configured to receive as input the reference data when generating the anesthesiology instructions.

5. The medical system of claim 2, 3, or 4, wherein the subject sensor system is any one of the following: a wearable device, a smart phone, a portable computing device, a vital sign measuring camera, a home medical system, and combinations thereof.

6. The medical system of any one of the preceding claims, wherein the subject physiological sensor data comprises any one of the following: ECG data, respiration rate, oxygen blood saturation level, heart rate, blood pressure, weight, galvanic skin conductance, and combinations thereof.

7. The medical system of any one of the preceding claims, wherein the medical system further comprises a robotic anesthesiology device (324), wherein execution of the local machine executable instructions further causes the computational system to output instructions adapted for controlling the robotic anesthesiology device using the anesthesiology instructions.

8. The medical system of any one of the preceding claims, wherein the medical system further comprises a magnetic resonance imaging system (502), wherein the anesthesiology instructions further comprise magnetic resonance imaging control commands configured to adjust acquisition of k-space data by the magnetic resonance imaging system, wherein the local memory further stores pulse sequence commands configured to control the magnetic resonance imaging system to acquire the k-space data, wherein execution of the local machine executable instructions further causes the local computational system to:
- adjust (600) the configuration of the magnetic resonance imaging system using the magnetic resonance imaging control commands;
- acquire (602) the k-space data by controlling the magnetic resonance imaging system with the pulse sequence commands.

9. The medical system of any one of any one of the preceding claims, wherein the medical system further comprises a local display (104) controlled by the local computational system, wherein execution of the local machine executable instructions further causes the local computational system to render (208) the anesthesiology instructions on the local display.

10. The medical system of any one of the preceding claims, wherein the control module comprises a database system indexed using a subject profile (334), wherein execution of the local machine executable instructions further causes the local computational system to:
- determine (412) the subject profile by applying a statistical algorithm to the subject metadata; and
- retrieve (414) the anesthesiology instructions from the database system using the subject profile.

11. The medical system of claim 10, wherein execution of the local machine executable instructions further causes the computational system to:
- receive (418) manual control commands (340) generated during execution of the anesthesiology instructions; and
- update (420) the anesthesiology instructions in the subject database with the manual control commands if the manual control commands differ from the anesthesiology instructions by more than a predetermined criterion.

12. The medical system of claim 10 or 11, wherein the statistical algorithm is further configured for detecting an out of distribution condition when being applied to the subject metadata and the subject physiological sensor data, and wherein execution of the local machine executable instructions further causes the local computational system to halt retrieval of the anesthesiology instructions if the out of distribution condition is detected.

13. The medical system of any one of the preceding claims,
wherein the subject metadata comprises any one of the following: social history, lifestyle data, demographic data, age, weight, type of procedure, electronic medical record data, exercise tolerance, cardiovascular fitness, respiratory input, oxygenation, ventilation, obtrusive sleep apnea, gastro-esophageal reflux, drugs/ medical records, anesthetic history, past surgical history, family history, genetic traits, and combinations thereof; and/or
wherein the anesthesiology instructions comprise a preoperative risk score and/or a postoperative risk score.

14. A method, wherein the method comprises:
- receiving (200) subject physiological sensor data via a network connection;
- receiving (202) subject metadata;
- receiving (204) anesthesiology instructions in response to inputting the subject physiological sensor data and the subject metadata into a control module, wherein the control module is configured to generate the anesthesiology instructions in response to receiving subject physiological sensor data and subject metadata as input; and
- providing (206) the anesthesiology instructions.

15. A computer program comprising local machine executable instructions and a control module for execution by a local computational system, wherein the control module is configured to generate anesthesiology instructions in response to receiving subject physiological sensor data and subject metadata as input, wherein execution of the local machine executable instructions causes the computational system to:
- receive (200) subject physiological sensor data via a network connection;
- receive (202) subject metadata;
- receive (204) the anesthesiology instructions in response to inputting the subject physiological sensor data and the subject metadata into the control module; and
- provide (206) the anesthesiology instructions.
